Europäisches Patentamt

European Patent Office

Office européen des brevets

(19)

(11) Veröffentlichungsnummer: **0 473 896 A2**

# EUROPÄISCHE PATENTANMELDUNG

(12)

(21) Anmeldenummer: **91110808.2**

(22) Anmeldetag: **29.06.91**

(51) Int. Cl.5: **G01N 33/00, G06F 15/00, G01N 1/24**

(30) Priorität: **01.09.90 DE 4027715**

(43) Veröffentlichungstag der Anmeldung:
**11.03.92 Patentblatt 92/11**

(84) Benannte Vertragsstaaten:
**AT BE CH DE DK ES FR GB GR IT LI LU NL SE**

(71) Anmelder: **S + G SCHMITT MESSGERÄTEBAU GMBH**
**Rheinhorststrasse 14**
**W-6700 Ludwigshafen(DE)**

(72) Erfinder: **Schmitt, Rudi**
**Rheinhorststrasse 14**
**W-6700 Ludwigshafen(DE)**
Erfinder: **Schmitt, Werner**
**Karl-Kreuter-Strasse 5**
**W-6700 Ludwigshafen(DE)**

(74) Vertreter: **Patentanwälte Zellentin & Partner**
**Rubensstrasse 30**
**W-6700 Ludwigshafen(DE)**

(54) **Messgerät, insbesondere zur Messung von Abgasen von Heizungsanlagen.**

(57) Die vorliegende Erfindung betrifft ein Meßgerät, insbesondere zur Messung von Abgasen von Heizungsanlagen, wobei in einem Meßkanal mindestens ein Sensor und eine Pumpe angeordnet sind, und zur Steuerung des Meßablaufs und zur Verarbeitung der Sensorsignale ein Mikrocomputer vorgesehen ist, der mit einer alphanumerischen Anzeigevorrichtung und zwei Bedienelementen verbunden ist, wobei ein Bedienelement jeweils zum Blättern in einem Auswahl-Menü dient, von dem jeweils ein Menüpunkt auf der Anzeigevorrichtung dargestellt ist, und wobei das andere Bedienelement zur Eingabe entsprechend dem jeweils dargestellten Menüpunkt dient.

Fig. 1

Rank Xerox (UK) Business Services

Die Erfindung betrifft ein Meßgerät, insbesondere zur Messung von Abgasen von Heizungsanlagen.

Zur Messung der Abgase von Heizungsanlagen sind verschiedene Meßgeräte bekannt, die jedoch recht umständlich zu handhaben und häufig recht schwer und groß sind. Es sind ferner Meßgeräte bekannt, die mit einem Computer arbeiten. Bei diesen ist jedoch durch die Vielzahl der Bedienelemente eine intensive Einarbeitung der Benutzer der Meßgeräte erforderlich.

Aufgabe der vorliegenden Erfindung ist es, ein Meßgerät vorzuschlagen, das einfach zu bedienen und das leicht zum bzw. am Einsatzort zu transportieren ist.

Das erfindungsgemäße Meßgerät ist dadurch gekennzeichnet, daß in einem Meßkanal mindestens ein Sensor und eine Pumpe angeordnet sind, daß zur Steuerung des Meßablaufs und zur Verarbeitung der Sensorsignale ein Mikrocomputer vorgesehen ist, der mit einer alphanumerischen Anzeigevorrichtung und zwei Bedienelementen verbunden ist, daß ein Bedienelement jeweils zum Blättern in einem Auswahl-Menü dient, von dem jeweils ein Menüpunkt auf der Anzeigevorrichtung dargestellt ist, und daß das andere Bedienelement zur Eingabe entsprechend dem jeweils dargestellten Menüpunkt dient.

Vorzugsweise ist dabei vorgesehen, daß die Bedienelemente Drucktasten sind und daß eine weitere Drucktaste mit einem Ein/Aus-Schalter verbunden ist.

Eine vorteilhafte Ausgestaltung besteht darin, daß die Drucktasten auf der Vorderseite des Meßgerätes, welche auch die Anzeigevorrichtung trägt, in der Nähe eines Randes der Vorderseite angeordnet sind. Dadurch kann das Meßgerät mit einer Hand gehalten und bedient werden. Die Gefahr von Fehlbedienungen wird vermindert, wenn die Drucktasten für das eine und das andere Bedienelement innerhalb einer Mulde näher beieinander angeordnet sind als die Drucktaste des Ein/Aus-Schalters.

Eine weitere Bedienungserleichterung wird mit einer Weiterbildung der Erfindung dadurch erzielt, daß nach einem für den Mikrocomputer vorgesehenen Programm die Folge der vom Bediener vorzunehmenden Eingaben und Messungen vorgegeben ist und daß Eingaben und Messungen nur möglich sind, wenn sie nach vorangegangenen Eingaben sinnvoll sind.

Eine andere Weiterbildung der Erfindung besteht darin, daß im Meßkanal ferner ein Drucksensor vorgesehen ist. Hierdurch wird eine einfache Messung des Kaminzuges möglich, wobei zusätzlich zur Aktivierung des entsprechenden Menüpunktes lediglich die Austrittsöffnung des Meßkanals zu verschließen ist.

Eine Messung verschiedener Temperaturen ist gemäß einer anderen Weiterbildung dadurch möglich, daß im Meßkanal ferner ein Temperatursensor angeordnet ist.

Durch die in weiteren Unteransprüchen aufgeführten Maßnahmen sind weitere vorteilhafte Weiterbildungen und Verbesserungen der im Hauptanspruch angegebenen Erfindung möglich.

Ausführungsbeispiele der Erfindung sind in der Zeichnung anhand mehrerer Figuren dargestellt und in der nachfolgenden Beschreibung näher erläutert. Es zeigt:

Fig. 1    eine Vorderansicht des Meßgerätes,
Fig. 2    eine Seitenansicht,
Fig. 3    eine Darstellung der zur Bedienung des Meßgerätes vorgesehenen Menüs,
Fig. 4    ein Blockschaltbild eines erfindungsgemäßen Meßgerätes,
Fig. 5    ein Flußdiagramm eines Unterprogramms und
Fig. 6    ein Flußdiagramm eines Programms für den Mikrocomputer bei dem Meßgerät nach Fig. 4.

Gleiche Teile sind in den Figuren mit gleichen Bezugszeichen versehen.

Das erfindungsgemäße Meßgerät weist ein quaderförmiges Gehäuse 1 auf. Die Vorderseite enthält ein Fenster 2 für eine Flüssigkristallanzeige 3, mit welcher in zwei Zeilen alphanumerische Zeichen dargestellt werden können. In der Nähe des rechten Randes der Vorderseite befindet sich zur Bedienung des Meßgerätes zwei Drucktasten 4, 5 und eine weitere Drucktaste 6 zum Ein- und Ausschalten des Meßgerätes. Die Drucktasten 4, 5 sind relativ nahe beieinander in einer Mulde 7 angeordnet, während die Drucktaste 6 etwas von den Drucktasten 4, 5 in einer separaten Mulde 8 abgesetzt ist. Durch die beschriebene Anordnung der Drucktasten wird ermöglicht, das Meßgerät mit der rechten Hand zu umfassen und die Drucktasten mit dem Daumen zu betätigen, wobei durch die größere Entfernung und die separate Mulde ein versehentliches Ausschalten des Meßgerätes erschwert wird.

An der linken Seitenwand 9 ist eine Ansaugöffnung 10 und eine Austrittsöffnung 11 für das jeweils zu messende Gas vorgesehen. In einem nicht dargestellten Meßkanal befinden sich eine Pumpe sowie Sensoren. Im einzelnen ist je eine elektrochemische Brennstoffzelle zur Messung von Sauerstoff und Kohlenmonoxid vorgesehen, während zur Temperaturmessung ein temperaturabhängiges Widerstandselement und zur Druckmessung ein piezoresistiver Druckwandler dient. Die Ausgangssignale der Sensoren werden in an sich bekannter Weise aufbereitet, analog/digital-gewandelt und einem Mikrocomputer zugeführt, in welchem unter anderem Programme zum Abgleich der einzelnen Sensoren, zur Fehlerkompensation und zur Entzerrung von gegebenenfalls nicht linearen Kennlinien der Sensoren zur Anwendung kommen.

An der linken Seitenwand 9 ist ferner ein Anschluß 12 für ein Stromversorgungsgerät vorgesehen, mit welchem auch der eingebaute Akkumulator aufgeladen werden kann. Ferner verfügt das Meßgerät über einen bügelförmigen Ständer 13, der aus dem Bereich der Rückwand ausgeklappt werden kann. Über eine nicht dargestellte Mehrfachbuchse, die eine genormte Schnittstelle, beispielsweise eine V24-Schnittstelle darstellt, können in dem Meßgerät gespeicherte Meßdaten ausgegeben werden - beispielsweise an einen Personalcomputer. An der Rückwand sind ferner Magnetbänder 14 zur Befestigung des Meßgerätes angeordnet, beispielsweise an einer Kesselwand.

Außer der bereits erwähnten Verarbeitung der Sensorsignale werden anhand geeigneter Programme vom Mikrocomputer noch weitere Funktionen erfüllt. So wird beispielsweise nach dem Einschalten ein Programm zur Justierung des Meßgerätes aktiviert. Dabei wird selbsttätig die Sauerstoffmeßeinrichtung auf 20,9 Volumen % kalibriert und die Kohlenmonoxideinrichtung auf 0 gestellt. Auch die zur Zugmessung dienende Druckmeßeinrichtung wird auf 0 justiert.

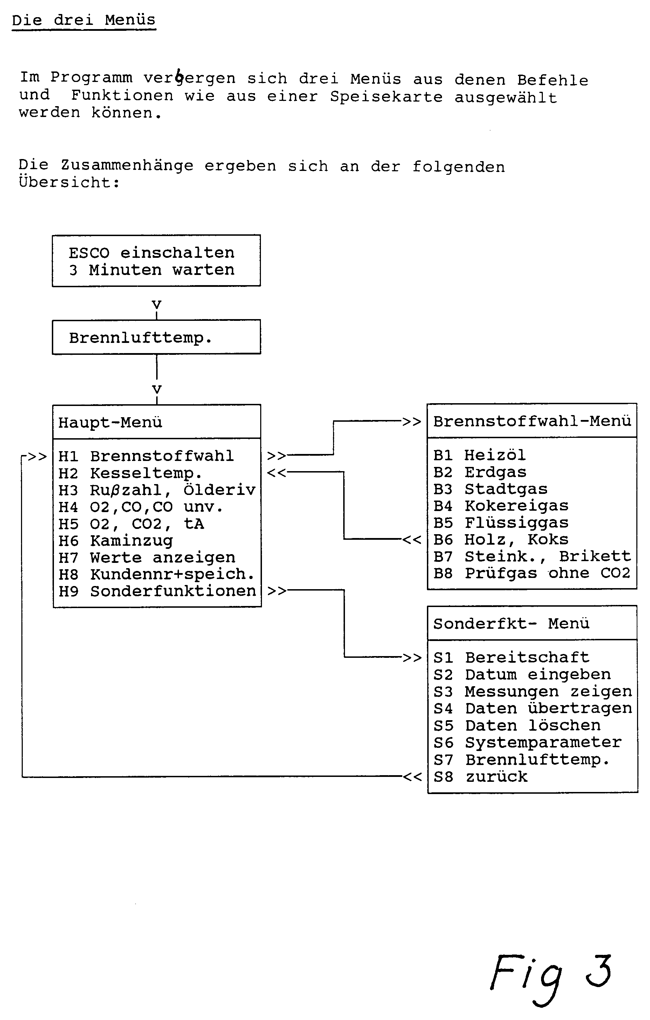

Die zum Betrieb des Meßgerätes erforderlichen Bedienvorgänge werden anhand von Fig. 3 erläutert. Nach dem Ablauf des Justierprogramms befindet sich das Programm des Mikrocomputers im Hauptmenü 21. Jeweils einer der Menüpunkte H1 bis H9 des Hauptmenüs 21 ist auf der Flüssigkristallanzeigevorrichtung - im folgenden LCD-Anzeige genannt - sichtbar. Nach dem Ablauf des Justierprogramms erscheint zunächst der Menüpunkt H1 "Brennstoffauswahl", da von dieser Auswahl alle weiteren Maßnahmen abhängen. Zur Auswahl des Brennstoffs wird die Eingabetaste 5 betätigt, worauf einer der Punkte B1 bis B6 des Brennstoffauswahlmenüs 22 in der LCD-Anzeige erscheint. Durch gegebenenfalls mehrmaliges Betätigen der Menü-Taste 4 wird der für den jeweiligen Brennstoff gültige Menüpunkt B1 bis B6 ausgewählt und anschließend mit der Eingabetaste 5 eingegeben. Mit Hilfe weiterer Menüpunkte des Hauptmenüs 21 können die jeweils für den ausgewählten Brennstoff erforderlichen Messungen durchgeführt werden.

Bei einem praktisch ausgeführten Meßgerät haben sich folgende weitere Menüpunkte bewährt:

H2 Kesseltemperatur,

H3 Eingabe der drei Rußzahlen und Ölderivate bei HEL,

H4 Messung und Anzeige von $O_2$, CO, CO unverdünnt (Abgaswegeüberprüfung mit Mehrlochsonde nur bei Gas),

H5 Messung und Anzeige von $O_2$, $CO_2$, Abgastemperatur (Abgasverlustmessung mit Kombisonde).

H6 Kaminzugmessung,

H7 Anzeige aller Meß- und Rechenwerte zur Kontrolle,

H8 Eingabe der Kundennummer und speichern, Start der nächsten Messung.

Wird die Eingabetaste 5 betätigt, wenn im Hauptmenü der Punkt H9 "Sonderfunktion" sichtbar ist, so wird ein Sonderfunktionsmenü 23 aufgerufen, mit welchen aus den Menüpunkten S1 bis S7 verschiedene Sonderfunktionen aufgerufen werden können. Mit dem Menüpunkt S8 gelangt der Benutzer wieder zurück in das Hauptmenü 21.

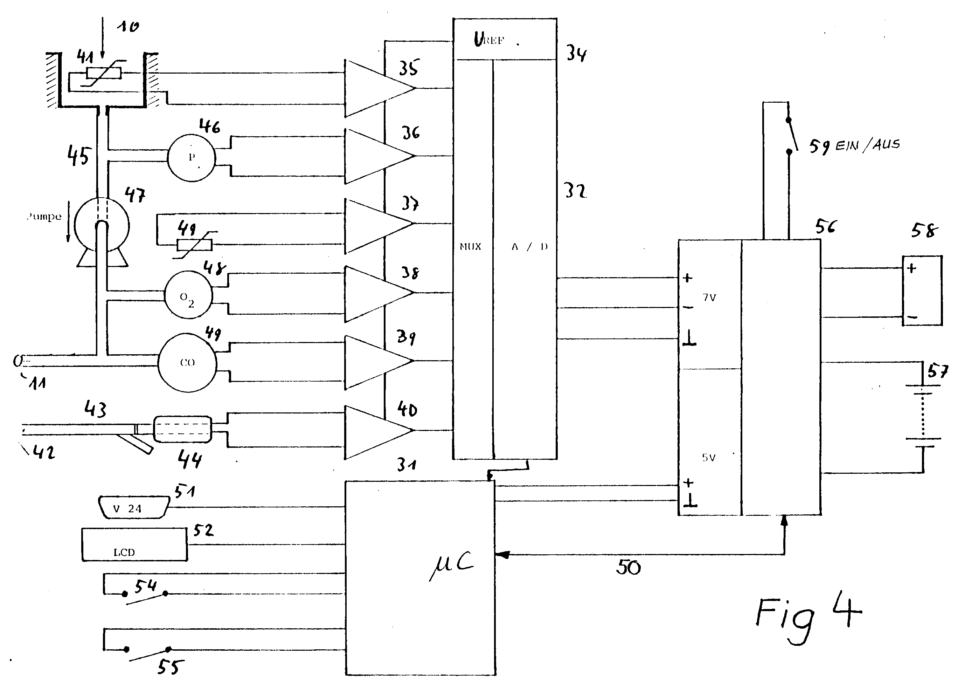

Fig. 4 zeigt ein Blockschaltbild eines erfindungsgemäßen Meßgerätes, bei welchem ein Mikrocomputer 31 in an sich bekannter Weise mit einem Analog/Digital-Wandler 32 verbunden ist, welchem ein Multiplexer 33 vorgeschaltet ist. Der Multiplexer 33 weist sechs Eingänge für Analogsignale auf. Dem Analog/Digital-Wandler 32 ist eine Referenzspannungsquelle 34 zugeordnet, die ferner mit Verstärkern 35 bis 40 in Verbindung steht. Diese dienen zur Verstärkung der von den Sensoren 41 bis 46 gelieferten Spannungen.

Ein erster Temperatursensor 41 befindet sich innerhalb der Ansaugöffnung 10 und dient zur Messung der Umgebungstemperatur. Ein zweiter Temperatursensor 42 befindet sich am Ende einer biegsamen Sonde 43, die wegen der elektrischen Verbindung mit dem Verstärker 40 dort dargestellt ist, jedoch mit einem entsprechenden Stecker 44 in die Ansaugöffnung eingesetzt werden kann.

Zur Messung des Drucks im Eingangsbereichs des Meßkanals 45 ist ein piezoelektrischer Drucksensor 46 vorgesehen. Eine Pumpe 47 pumpt das zu messende Gas von der Ansaugöffnung 10 des Gaskanals zur Austrittsöffnung 11. Da der Drucksensor 46 im Eingangsbereich angeordnet ist, ist eine einfache Messung des Drucks, insbesondere des Kaminzugs dadurch möglich, daß einerseits der entsprechende Menüpunkt aufgerufen wird und andererseits die Austrittsöffnung 11 verschlossen wird.

Im weiteren Verlauf des Meßkanals 45 sind Sensoren 48, 49 zur Messung von Sauerstoff und Kohlenmonoxid angeordnet. Geeignete Sensoren in Form von elektro-chemischen Brennstoffzellen sind im Handel erhältlich. Um die Temperaturabhängigkeit des Sauerstoffsensors 48 kompensieren zu können, wird die Temperatur des Sauerstoffsensors 48 mit Hilfe eines dritten Temperatursensors 49 gemessen.

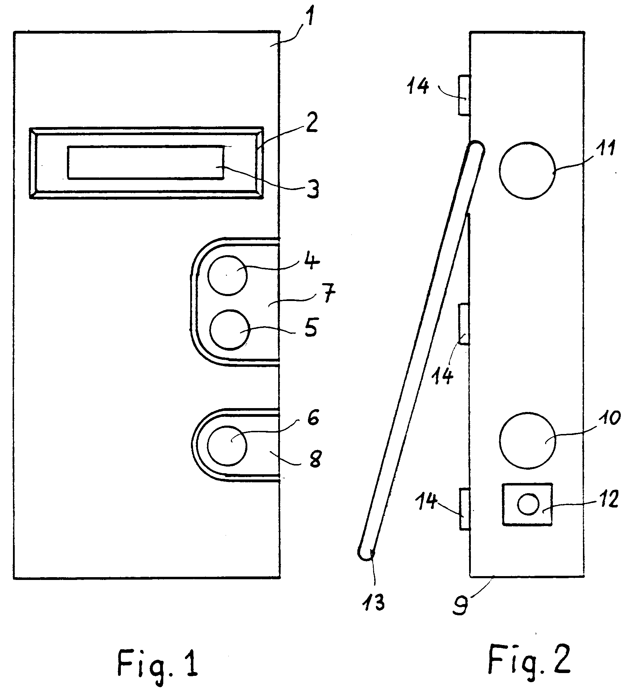

Die Ausgangssignale der Sensoren werden nach entsprechender Verstärkung mit Hilfe des Multiplexers 33 und des Analog/Digital-Wandlers 32 in ein digitales Multiplexsignal umgewandelt, das dem Mikrocomputer 31 zugeführt wird. An geeignete Ein/Ausgänge des Mikrocomputers 31 sind ferner angeschlossen: eine V24-Schnittstelle 51, eine Flüssigkristallanzeige 52 und Kontakte 54, 55 der Drucktasten 4, 5 (Fig. 1).

Eine Stromversorgungsschaltung 56 erzeugt aus der Spannung eines Akkumulators 57 oder aus einer bei 58 zugeführten Gleichspannung eine Betriebsspannung von 5V für den Mikrocomputer 31 und eine Betriebsspannung von 7V für den Analog/Digital-Wandler 32 und den Multiplexer 33. Mit der Stromversorgungsschaltung 56 sind ferner Kontakte 59 der Ein/Aus-Taste 6 (Fig. 1) verbunden. Außerdem kann mit Hilfe der Stromversorgungsschaltung 56 der Akkumulator 57 über die bei 58 zugeführte Gleichspannung geladen werden. Der Mikrocomputer 31 und die Stromversorgungsschaltung 56 sind durch eine Datenleitung 50 miteinander verbunden. Dadurch kann für den Fall des Abschaltens des Geräts vom Mikrocomputer 31 die Stellung der Kontakte 59 abgefragt werden. Ergibt die Abfrage, daß die Drucktaste 6 betätigt wurde, veranlaßt der Mikrocomputer 31 über die Datenleitung 50 das Abschalten des Geräts. Je nach Betriebszustand kann dieses sofort nach der Tastenbetätigung oder später erfolgen - beispielsweise nachdem die gemessenen Daten abgespeichert sind.

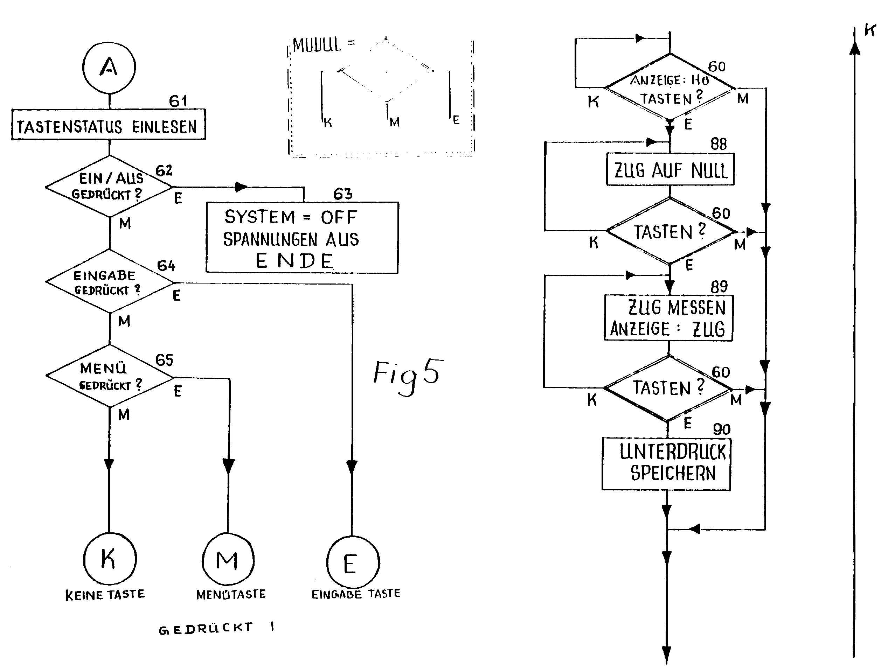

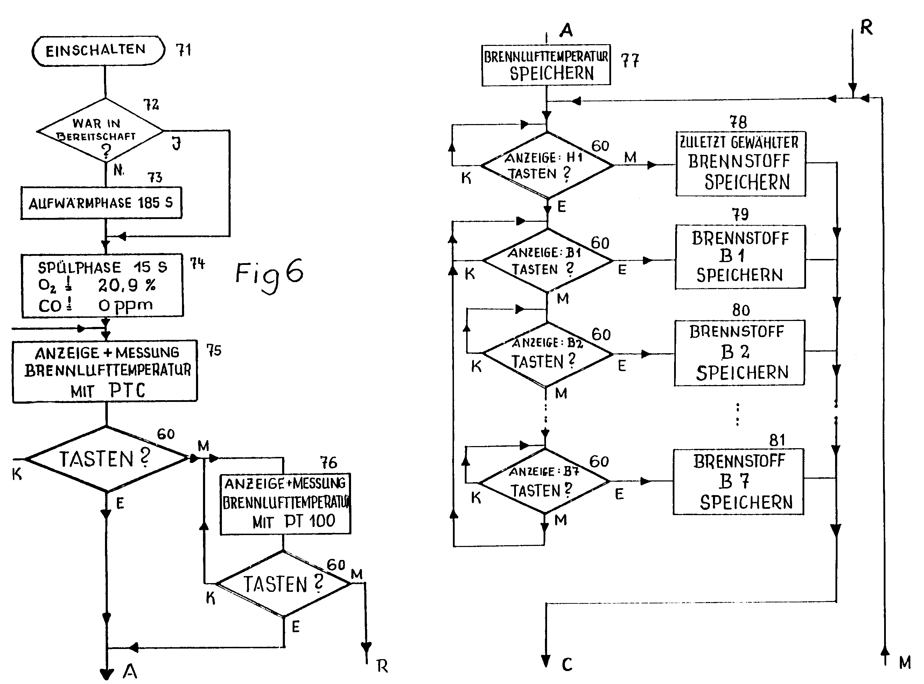

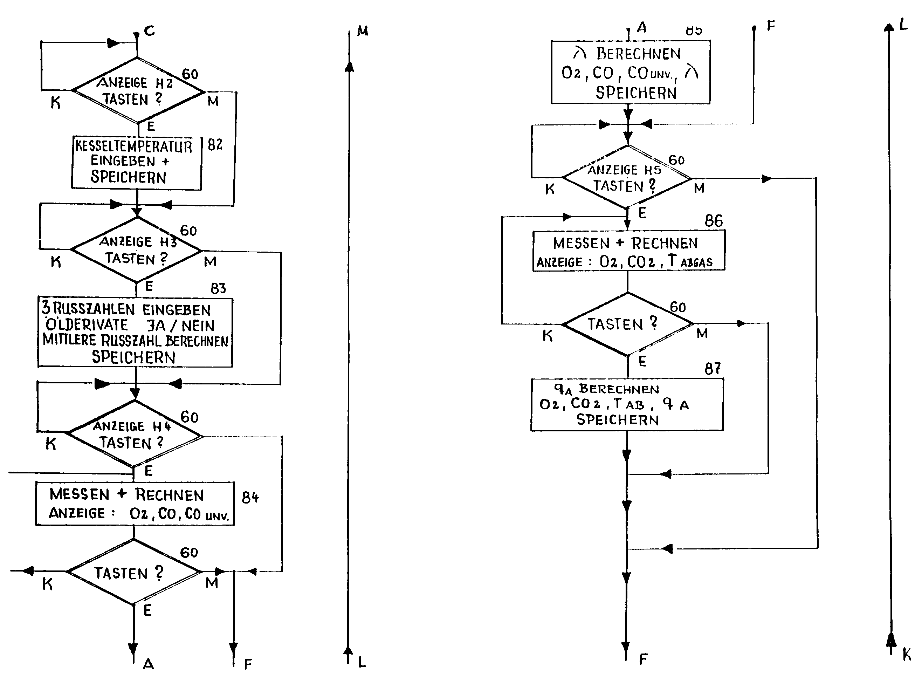

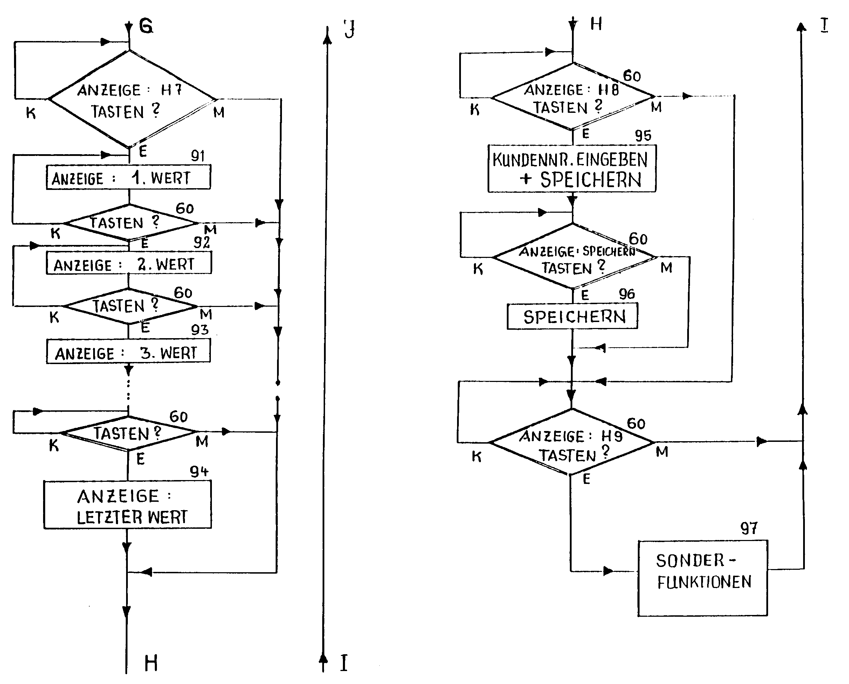

Fig. 5 zeigt in Form eines Flußdiagramms ein Unterprogramm zur Abfrage der Drucktasten 4, 5, 6 (Fig. 1), das bei dem in Fig. 6 dargestellten Flußdiagramm wiederholt aufgerufen wird. Nach dem Start des Unterprogramms bei A wird im Programmteil 61 der Tastenstatus eingelesen. Danach verzweigt sich bei 62 das Unterprogramm in Abhängigkeit davon, ob die Ein/Aus-Taste gedrückt ist. Ist dieses der Fall, wird bei 63 das Gerät abgeschaltet. Ist die Ein/Aus-Taste 6 nicht gedrückt, wird das Programm bei 64 in Abhängigkeit davon verzweigt, ob die Eingabetaste 5 gedrückt ist. Wenn ja, wird das Unterprogramm bei E verlassen, wenn nicht, wird in einer weiteren Verzweigung 65 gefragt, ob die Menütaste 4 gedrückt ist. Ist dieses der Fall, wird das Unterprogramm bei M verlassen; anderenfalls endet das Unterprogramm bei K.

Das in Fig. 5 dargestellte Unterprogramm ist in Fig. 6 mit dem Bezugszeichen 60 versehen, wobei jedoch der Übersichtlichkeit halber der Ausgang des Unterprogramms, der ein Abschalten des Gerätes zur Folge hat, nicht dargestellt ist.

Nach dem Einschalten bei 71 wird bei dem in Fig. 6 dargestellten Programm zunächst bei 72 geprüft, ob das Gerät sich zuvor in einer Betriebsbereitschaft befunden hat. Ist dieses nicht der Fall, wird bei 73 für eine Zeit von 185s eine Aufwärmphase durchgeführt. Im Programmteil 74 folgt eine Spülphase von 15s, bei welcher die Pumpe 47 (Fig. 4) in Betrieb genommen wird und die dann vorliegenden Meßergebnisse der Sensoren 48, 49 zu 20,9 % und 0 ppm eingestellt werden.

Das Gerät ist jetzt bereit zur Messung der Brennlufttemperatur mit dem zweiten Temperaturfühler 42 (Fig. 4). Dieses erfolgt im Programmteil 75. Darauf folgt eine Tastenabfrage 60. Solange keine Taste gedrückt ist, bleibt es bei der Anzeige und Messung der Brennlufttemperatur. Ist jedoch die Menütaste gedrückt, erfolgt bei 76 eine Messung und Anzeige der Brennlufttemperatur mit dem im Eingangsbereich des Meßkanals 45 befindlichen Sensor 41. Wird jedoch während des Unterprogramms 60 die Eingabetaste gedrückt, wird bei 77 der bei 75 gemessene Wert der Brennlufttemperatur gespeichert, das heißt, in einem Schreib/Lese-Speicher des Mikrocomputers 31 (Fig. 4) abgelegt. Wird nach dem Programmteil 76 die Eingabetaste gedrückt, so wird der im Programmteil 76 gemessene Wert der Brennlufttemperatur gespeichert.

Wird jedoch nach dem Programmteil 76 die Menütaste gedrückt, so wird das Programm ohne Speichern der Brennlufttemperatur mit dem Unterprogramm 60 fortgesetzt, wobei auf der Flüssigkristallanzeige 52 (Fig. 4) die Zeile H1 des Hauptmenüs 21 (Fig. 3) dargestellt wird. Wird dann nochmals die Menütaste gedrückt, so werden für den zuletzt gewählten Brennstoff Daten gespeichert. Wird im Unterprogramm 60 jedoch die Eingabetaste gedrückt, so wird das Unterprogramm 60 noch einmal aufgerufen, jedoch mit der Zeile B1 des Brennstoffwahl-Menüs 22 (Fig. 3). Durch Betätigen der Menütaste kann das Unterprogramm 60 mit einer Anzeige einer anderen Zeile des Brennstoffwahl-Menüs wiederholt werden, bis der gewünschte Brennstoff ausgewählt ist. Der Übersichtlichkeit halber sind in Fig. 6 nicht alle Wiederholungen dargestellt. Nach Drücken der Eingabetaste bei dem jeweiligen Lauf des Unterprogramms 60 werden die Daten des dann ausgewählten Brennstoffs in einem der Programmteile 79 bis 81 in den Schreib/Lese-Speicher des Mikrocomputers 31 übernommen.

Nach dieser Brennstoffauswahl wird die Zeile H2 des Hauptmenüs 21 angezeigt. Es wird somit zu einer erneuten Tasteneingabe aufgefordert. Wird die Eingabetaste gedrückt, wird im Programmteil 82 die Kesseltemperatur eingegeben und abgespeichert. Danach erfolgt die Anzeige der Zeile H3 des Hauptmenüs und eine erneute Tasteneingabe. Wird in diesem Fall die Eingabetaste gedrückt, können mit Hilfe eines im einzelnen nicht dargestellten Untermenüs im Programmteil 83 drei Rußzahlen eingegeben und die Frage beantwortet werden, ob Öldevirate berücksichtigt werden sollen oder nicht. In diesem Programmteil wird dann die mittlere Rußzahl berechnet und gespeichert.

Anschließend erfolgt in dem Unterprogramm 60 die Anzeige der Zeile H4 des Hauptmenüs und eine Tasteneingabe, welche in diesem Fall nur in der Betätigung der Eingabetaste bestehen kann. Daraufhin erfolgen der Meßvorgang und die Auswertung der gemessenen Größen und die Anzeige des Sauerstoffgehalts, des Kohlenmonoxidgehalts und des unverdünnten Kohlenmonoxidgehalts im Programmteil 84. Bei einer folgenden Tasteneingabe wird dann entschieden, ob lambda berechnet werden soll. Ist dieses der Fall, so werden die Werte für lambda, Sauerstoff und Kohlenmonoxid abgespeichert (Programmteil 85).

Danach wird die Zeile H5 des Hauptmenüs angezeigt und auf eine Tasteneingabe gewartet. Wird die Eingabetaste betätigt, so wird im Programmteil 86 nochmals gemessen und gerechnet und es werden die Werte für Sauerstoff, Kohlendioxid und die Abgastemperatur angezeigt. Nach erneuter Betätigung der Eingabetaste wird im Programmteil 87 $q_A$ berechnet und zusätzlich zu den oben genannten Werten gespeichert.

In den folgenden Programmteilen wird eine Messung des Kaminzugs vorgenommen, wenn nach Anzeige der Zeile H6 des Hauptmenüs die Eingabetaste gedrückt wird. Nach einer Betätigung der Eingabetaste im Unterprogramm 60 wird bei 88 der Zug auf 0 gestellt. Danach ist nochmals die Eingabetaste zu betätigen, worauf im Programmteil 89 der Zug gemessen und angezeigt wird. Nach nochmaliger Betätigung der Eingabetaste wird bei 90 der im Kaminzug repräsentierende Unterdruck gespeichert.

Die folgenden Programmteile dienen dazu, die bei den bisher erfolgten Messungen verwendeten Parameter und gemessenen sowie berechneten Werte anzuzeigen, wenn nach Anzeige der Zeile H6 des Hauptmenüs die Eingabetaste gedrückt wird. Dabei wird jeweils ein Teil dieser Angaben in den Programmteilen 91 bis 94 angezeigt und dazwischen mit Hilfe des Unterprogramms 60 dem Benutzer die Möglichkeit gegeben, entweder sich den nächsten Wert anzeigen zu lassen oder ins Hauptmenü zurückzukehren.

Danach wird in einem Unterprogramm 60 die Zeile H8 des Hauptmenüs angezeigt, in der zur Eingabe der Kundennummer aufgefordert wird. Soll dieses geschehen, wird nach Betätigung der Eingabetaste ein Programm 95 aufgerufen, bei welchem wiederum in Form eines Untermenüs dezimalstellenweise je eine Ziffer mit Hilfe der Menütaste und der Eingabetaste ausgewählt wird. Danach wird mit Hilfe des Unterprogramms 60 gefragt, ob zu der bei 95 gewählten Kundennummer die gemessenen Daten gespeichert werden sollen. Zutreffendenfalls erfolgt die Speicherung im Programmteil 96.

Im Anschluß daran wird wieder das Unterprogramm 60 aufgerufen und die Menüzeile H9 angezeigt, welche besagt, ob ein Sonderfunktionsmenü 23 (Fig. 3) aktiviert werden soll. Ist dieses nicht der Fall, kehrt das Programm nach Betätigung der Menütaste zur Anzeige der Zeile H1 des Hauptmenüs zurück. Anderenfalls wird ein Programm 97 mit Sonderfunktionen aufgerufen, welches verschiedene weitere Funktionen, beispielsweise die Übertragung der gespeicherten Daten über die V24-Schnittstelle und Möglichkeiten zur Verwaltung der gespeicherten Daten, vorsieht.

## Patentansprüche

1. Meßgerät, insbesondere zur Messung von Abgasen von Heizungsanlagen, dadurch gekennzeichnet, daß in einem Meßkanal mindestens ein Sensor und eine Pumpe angeordnet sind, daß zur Steuerung des Meßablaufs und zur Verarbeitung der Sensorsignale ein Mikrocomputer vorgesehen ist, der mit einer alphanumerischen Anzeigevorrichtung (3) und zwei Bedienelementen (4, 5) verbunden ist, daß ein Bedienelement (4) jeweils zum Blättern in einem Auswahl-Menü dient, von dem jeweils ein Menüpunkt auf der Anzeigevorrichtung (3) dargestellt ist, und daß das andere Bedienelement (5) zur Eingabe entsprechend dem jeweils dargestellten Menüpunkt dient.

2. Meßgerät nach Anspruch 1, dadurch gekennzeichnet, daß die Bedienelemente Drucktasten (4, 5) sind und daß eine weitere Drucktaste (6) mit einem Ein/Aus-Schalter verbunden ist.

3. Meßgerät nach Anspruch 2, dadurch gekennzeichnet, daß die Drucktasten (4, 5, 6) auf der Vorderseite des Meßgerätes, welche auch die Anzeigevorrichtung (3) trägt, in der Nähe eines Randes der Vorderseite angeordnet sind.

4. Meßgerät nach Anspruch 3, dadurch gekennzeichnet, daß die Drucktasten (4, 5) für das eine und das andere Bedienelement innerhalb einer Mulde (7) näher beieinander angeordnet sind als die Drucktaste (6) des Ein/Aus-Schalters.

5. Meßgerät nach einem der vorhergehenden Ansprüche, dadurch gekennzeichnet, daß nach einem für den Mikrocomputer vorgesehenen Programm die Folge der vom Bediener vorzunehmenden Eingaben und Messungen vorgegeben ist und daß Eingaben und Messungen nur möglich sind, wenn sie nach vorangegangenen Eingaben sinnvoll sind.

6. Meßgerät nach einem der vorhergehenden Ansprüche, dadurch gekennzeichnet, daß nach dem Einschalten eine im Meßgerät vorhandene Sauerstoffmeßeinrichtung auf 20,9 Volumen % und eine im Meßgerät vorhandene CO-Meßeinrichtung auf 0 kalibriert wird.

7. Meßgerät nach einem der vorhergehenden Ansprüche, dadurch gekennzeichnet, daß im Meßkanal ferner ein Drucksensor vorgesehen ist.

8. Meßgerät nach Anspruch 7, dadurch gekennzeichnet, daß vor der Druckmessung der Drucksensor auf 0 hPa kalibriert wird.

9. Meßgerät nach einem der vorhergehenden Ansprüche, dadurch gekennzeichnet, daß im Meßkanal ferner ein Temperatursensor angeordnet ist.

10. Meßgerät nach einem der vorhergehenden Ansprüche, dadurch gekennzeichnet, daß eine Ansaugöffnung (10) und eine Austrittsöffnung (11) des Meßkanals vorgesehen sind, wobei mindestens die Ansaugöffnung an der von den Bedienelementen (4, 5) entfernten Seite des Meßgerätes angeordnet ist.

11. Meßgerät nach Anspruch 10, dadurch gekennzeichnet, daß eine Sondenleitung an die Ansaugöffnung (10) anschließbar ist.

12. Meßgerät nach einem der vorhergehenden Ansprüche, dadurch gekennzeichnet, daß an der Rückseite vorzugsweise flexible Magnete (14) angeordnet sind.

13. Meßgerät nach einem der vorhergehenden Ansprüche, dadurch gekennzeichnet, daß ein Stecker zur Übertragung von Daten vorgesehen ist.

Fig. 1

Fig. 2

## Die drei Menüs

Im Programm verbergen sich drei Menüs aus denen Befehle
und Funktionen wie aus einer Speisekarte ausgewählt
werden können.

Die Zusammenhänge ergeben sich an der folgenden
Übersicht:

```
┌─────────────────────┐
│  ESCO einschalten   │
│  3 Minuten warten   │
└─────────────────────┘
           v
┌─────────────────────┐
│  Brennlufttemp.     │
└─────────────────────┘
           v
┌─────────────────────┐              >>  ┌─────────────────────────┐
│  Haupt-Menü         │                  │  Brennstoffwahl-Menü    │
├─────────────────────┤  >>──┐           ├─────────────────────────┤
│ H1  Brennstoffwahl  │  <<──┘           │  B1  Heizöl             │
│ H2  Kesseltemp.     │                  │  B2  Erdgas             │
│ H3  Rußzahl, Ölderiv│                  │  B3  Stadtgas           │
│ H4  O2,CO,CO unv.   │                  │  B4  Kokereigas         │
│ H5  O2, CO2, tA     │                  │  B5  Flüssiggas         │
│ H6  Kaminzug        │              <<  │  B6  Holz, Koks         │
│ H7  Werte anzeigen  │                  │  B7  Steink., Brikett   │
│ H8  Kundennr+speich.│                  │  B8  Prüfgas ohne CO2   │
│ H9  Sonderfunktionen│  >>──┐           └─────────────────────────┘
└─────────────────────┘                  ┌─────────────────────────┐
                                         │  Sonderfkt- Menü        │
                                   >>    ├─────────────────────────┤
                                         │  S1  Bereitschaft       │
                                         │  S2  Datum eingeben     │
                                         │  S3  Messungen zeigen   │
                                         │  S4  Daten übertragen   │
                                         │  S5  Daten löschen      │
                                         │  S6  Systemparameter    │
                                         │  S7  Brennlufttemp.     │
                                   <<    │  S8  zurück             │
                                         └─────────────────────────┘
```

*Fig 3*

Fig 4

EP 0 473 896 A2

A

TASTENSTATUS EINLESEN — 61

EIN / AUS GEDRÜCKT ? — 62
E
M

SYSTEM = OFF
SPANNUNGEN AUS
E N D E — 63

EINGABE GEDRÜCKT ? — 64
E
M

MENÜ GEDRÜCKT ? — 65
E
M

K
KEINE TASTE

M
MENÜTASTE

E
EINGABE TASTE

GEDRÜCKT !

MODUL =
K   M   E

Fig 5

ANZEIGE: H∅ TASTEN ? — 60
K   M   E

ZUG AUF NULL — 88

TASTEN ? — 60
K   M   E

ZUG MESSEN
ANZEIGE : ZUG — 89

TASTEN ? — 60
K   M   E

UNTERDRUCK SPEICHERN — 90

K

10

EINSCHALTEN 71

72 WAR IN BEREITSCHAFT ? J

N. 73 AUFWÄRMPHASE 185 S

Fig 6

SPÜLPHASE 15 S 74
O₂ ↓ 20,9 %
CO ↓ 0 ppm

ANZEIGE + MESSUNG 75
BRENNLUFTTEMPERATUR
MIT PTC

TASTEN ? 60 M

K E

ANZEIGE + MESSUNG 76
BRENNLUFTTEMPERATUR
MIT PT 100

TASTEN ? 60 M
K E

A R

BRENNLUFTTEMPERATUR 77
SPEICHERN

A R

ANZEIGE: H1 60 M ZULETZT GEWÄHLTER 78
TASTEN ? BRENNSTOFF
K E SPEICHERN

ANZEIGE: B1 60 BRENNSTOFF 79
TASTEN ? E B1
K M SPEICHERN

ANZEIGE: B2 60 BRENNSTOFF 80
TASTEN ? E B2
K M SPEICHERN

ANZEIGE: B7 60 BRENNSTOFF 81
TASTEN ? E B7
K M SPEICHERN

C M

11

ANZEIGE H2
TASTEN ?
60
K
E
M

KESSELTEMPERATUR
EINGEBEN +
SPEICHERN
82

ANZEIGE H3
TASTEN ?
60
K
E
M
83

RUSSZAHLEN EINGEBEN
ÖLDERIVATE 3A / NEIN
MITTLERE RUSSZAHL BERECHNEN
SPEICHERN

ANZEIGE H4
TASTEN ?
60
K
E
M

MESSEN + RECHNEN
ANZEIGE : O₂, CO, CO unv.
84

TASTEN ?
60
K
E
M

λ BERECHNEN
O₂, CO, CO unv, λ
SPEICHERN
85

ANZEIGE H5
TASTEN ?
60
K
E
M
86

MESSEN + RECHNEN
ANZEIGE : O₂, CO₂, T ABGAS

TASTEN ?
60
K
E
M
87

qₐ BERECHNEN
O₂, CO₂, T AB, qₐ
SPEICHERN

ANZEIGE: LETZTER WERT 94

TASTEN? 60 — K E M

ANZEIGE: 3. WERT 93

TASTEN? 60 — K E M

ANZEIGE: 2. WERT 92

TASTEN? 60 — K E M

ANZEIGE: 1. WERT 91

ANZEIGE: H7 TASTEN? 60 — K E M

H  I  J  G

ANZEIGE: H9 TASTEN? 60 — K E M

SONDER-FUNKTIONEN 97

SPEICHERN

ANZEIGE: SPEICHERN TASTEN? 60 96 — K E M

KUNDENNR. EINGEBEN + SPEICHERN 95

ANZEIGE: H8 TASTEN? 60 — K E M

H  I